(19)

Europäisches
Patentamt
European
Patent Office
Office européen
des brevets

(11) **EP 3 045 155 A1**

(12) **EUROPEAN PATENT APPLICATION**

(43) Date of publication:
**20.07.2016 Bulletin 2016/29**

(51) Int Cl.:
*A61F 7/00* (2006.01)　　　*G06F 19/00* (2011.01)

(21) Application number: **15151523.6**

(22) Date of filing: **16.01.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **ProCcare io BVBA
2980 Zoersel (BE)**

(72) Inventors:
• **Viroux, Patrick
2980 Zoersel (BE)**
• **Tiemessen, Ivo
1040 GE Amsterdam (NL)**

(74) Representative: **Pappaert, Kris et al
De Clercq & Partners
Edgard Gevaertdreef 10 a
9830 Sint-Martens-Latem (BE)**

(54) **Personalized thermal treatment protocols**

(57) The present invention relates to methods and devices for the personalization of thermal treatment protocols. In particular, standard thermal treatment protocols are adapted and personalized based on individual-specific information, allowing more effective treatment protocols. Also systems for automated planning of the thermal treatment of an individual or a part thereof with individual-specific consideration are provided.

EP 3 045 155 A1

Printed by Jouve, 75001 PARIS (FR)

**Description**

**FIELD OF THE INVENTION**

The present invention relates to methods and devices for personalizing thermal treatment protocols. In particular, standard thermal treatment protocols are adapted and personalized based on individual-specific information, allowing more effective treatment protocols. Also systems for automated planning of the thermal treatment of an individual or a part thereof with individual-specific consideration are provided.

**BACKGROUND**

Particular ailments, injuries, or diseases require treatment involving cold and/or heat application to a body part, which has a particular beneficial medical outcome, according to the treatment protocol applied (e.g. intensity and duration of treatment). Other afflictions or injuries require treatment involving cold and/or heat application to an individual's entire body. Also, the application of cold or heat to an individual or a part thereof is commonly used for the treatment of minor pains and aches in athletes during recovery and after intensive exercise. Another field where the application of cold or heat to a body part is of great importance is the recovery phase after major surgical interventions or medical treatments as well as the recovery phase during conservative treatment and rehabilitation.

In the past, thermal treatments were accomplished by, for example, applying ice packs to the affected area for cryotherapy, and applying a heat source, such as a heated towel, for heat therapy. However, it is often seen that the application of cold or heat is performed too short, or that the application of cold or heat is performed using the wrong temperature thresholds, thereby rendering the treatment less effective. Often the temperature of ice packs is too low and the person to whom it is applied will not sustain the thermal treatment for a sufficiently long period for the treatment to be effective.

Recently, in the technical field of therapeutic treatment of a human or animal body by means of a thermal treatment, systems have been developed which provide circulation of fluids through pads that can be attached to a body part, the system being able to actively control and regulate the pads' temperature. Particularly, the treatment of the body part is carried out through controlled and actively regulated thermal energy exchange between the pad and the body part to which it is applied.

Apart from localized heat-or cold application, whole body application of heat or cold is also known in the State-of-the-Art. For example, whole body application of heat or cold may be administered by means of bath tubs or whirlpool baths. Whirlpool baths can combine the beneficial effects of thermotherapy and massage using water jets. Alternatively, whole body application of heat may be administered by means of saunas. For example, sauna's can, in combination with the use of cold showers afterwards, be useful to alleviate rheumatic pain. Also, whole body application of cold may be administered by means of cryogenic chambers. An individual treated in a cryogenic chamber is exposed to very cold temperatures for a short time. Typically, the exposure occurs at temperatures ranging from -110 to -160 °C for no more than three minutes. Typically, a cryogenic chamber is cooled with liquid nitrogen. Treated individuals are protected from acute frostbite by wearing a bathing suit, mouth and ear protection, socks, and gloves. After a few minutes of treatment, the surface skin temperature drops significantly, but the core body temperature remains mostly unchanged during the treatment. The uses of cryogenic chamber treatments include assisting athletes recover after an intensive training. Note that the term "thermal treatment" as used herein may refer to either local- or whole-body application of cold or heat, unless indicated otherwise.

At present, thermal treatments directed to individuals are not standardized nor personalized. Typically every athlete or patient is thermally treated in the same way irrespective of the individual's personal characteristics, thereby often rendering the treatment either too severe or less effective.

When the thermal treatment is too severe, the person undergoing the treatments will often feel discomfort and consequently stop the thermal treatment, thereby jeopardizing the revalidation. When the thermal treatment does not achieve the required temperatures at the body part to be treated, the treatment is rendered less effective, thereby also jeopardizing the therapy's effectiveness.

**SUMMARY OF THE INVENTION**

In a first aspect, the present invention comprises a method for the personalized planning of the thermal treatment of an individual, the method comprising:

- providing a standard thermal treatment protocol adapted to the condition of said individual;
- receiving individual-specific information;
- applying said individual-specific information to said standard thermal treatment protocol, thereby adjusting parameters of said standard thermal treatment protocol such as the duration, number of intervals and/or intensity of the standard thermal treatment protocol; and

- outputting the adjusted thermal treatment protocol as the personalized protocol for the thermal treatment of said individual.

In particular embodiments, the individual-specific information comprises general information chosen from age, gender, mass, height, fitness or a combination thereof as well as individual-specific anatomical information such as subcutaneous fat content of the treated body part, the depth of the injury or a combination thereof.

In particular embodiments, the subcutaneous fat content of the treated body part is calculated based on the general information.

In particular embodiments the individual-specific information is obtained from anatomy measurements of the individual.

In particular embodiments, the standard thermal treatment protocol is a generic thermal treatment protocol function of medical knowledge collected from literature converted into a functional thermal treatment protocol, preferably based on for instance the anatomical region to be treated, the structure, the recovery phase, the depth of the injury, the level on severity of the injury or a combination thereof.

In particular embodiments, applying said individual-specific information to said standard thermal treatment protocol results in an adaptation of the temperature parameters, duration of the temperature intervals, frequency of the temperature intervals, the frequency of the cycles of thermal treatment, the slope of temperature changes, the overall duration of the protocol temperature intervals or a combination thereof.

In particular embodiments, the adjusted thermal treatment protocol is evaluated prior to outputting the adjusted thermal treatment protocol to monitor whether the adjusted parameters do not exceed pre-set thresholds.

In particular embodiments, the thermal treatment protocol comprises an inslope phase, a treatment phase and an outslope phase. In particular embodiments the treatment phase comprises temperature modulations.

In particular embodiments, the personalized thermal treatment protocol is executed on the individual once the personalized thermal treatment protocol has been determined for the individual,

In particular embodiments, a method according to the present invention comprises receiving information from sensors on the skin of said individual during the personalized thermal treatment of the individual and applying said information to the personalized thermal treatment protocol, thereby further adjusting said personalized thermal treatment protocol.

In a second aspect, the present invention comprises a computer program product comprising one or more computer readable media having computer executable instructions for performing the steps of the method according to the first aspect of the present invention and/or any particular embodiments thereof.

In a third aspect, the present invention comprises a system for the automated planning of the thermal treatment of an individual or a part thereof with individual-specific consideration, the system comprising

- a thermal treatment protocol database which stores one or more standard thermal treatment protocols; said standard thermal treatment protocols being generic thermal treatment protocols in function of medical knowledge collected from literature converted into a thermal treatment protocol, preferably based on for instance the anatomical region to be treated, the structure, the recovery phase, the depth of the injury, the level on severity of the injury or a combination thereof;

- one or more processors programmed to receive a plurality of individual-specific parameters of said individual or a part thereof; and information about the required thermal treatment for said individual or a part thereof;

said one or more processors being operable to apply to a standard thermal treatment protocol the individual-specific parameters thereby adjusting parameters of said standard thermal treatment protocol such as the duration, number of intervals and/or intensity of the standard thermal treatment protocol and generating an adjusted thermal treatment protocol as the personalized protocol for the thermal treatment of said individual or a part thereof; and; said one or more processors being operable to display the personalized protocol for the thermal treatment of said individual.

In particular embodiments, the system further comprises an input operable to receive said individual-specific parameters of said individual; and the information about the required thermal treatment for said individual.

In particular embodiments, the system further comprises measurement means adapted to acquire data of a thermal treatment from the individual; and means for adapting the thermal treatment protocol of the individual on the basis of the data acquired.

In particular embodiments, the system further comprises one or more thermal modalities chosen from the list consisting of: hydraulic devices, Peltier thermal exchange elements, water immersion facilities, bath tubs, compressor cooling devices, vapor-compression refrigeration devices and cryogenic chambers.

BRIEF DESCRIPTION OF THE DRAWINGS

The following description of the figures of specific embodiments of the invention is merely exemplary in nature and is not intended to limit the present teachings, their application or uses. Throughout the drawings, corresponding reference numerals indicate like or corresponding parts and features.

Figure 1 shows three different thermal treatment protocols for an upper leg injury. Protocol (1) is a reference treatment protocol, protocol (2) is a treatment protocol for a first individual, and protocol (3) is a treatment protocol for a third individual.

Figure 2 shows three different thermal treatment protocols for an acute, level 2, deep, lower leg muscle injury. Protocol (4) is a reference treatment protocol, protocol (5) is a treatment protocol for a first individual, and protocol (6) is a treatment protocol for a third individual.

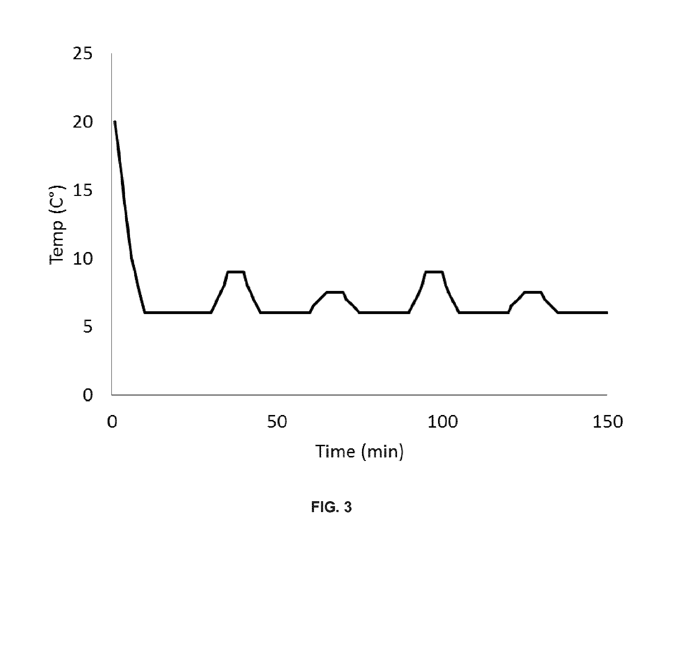

Figure 3 shows a reference treatment protocol for thermal treatment of an acute level three joint injury.

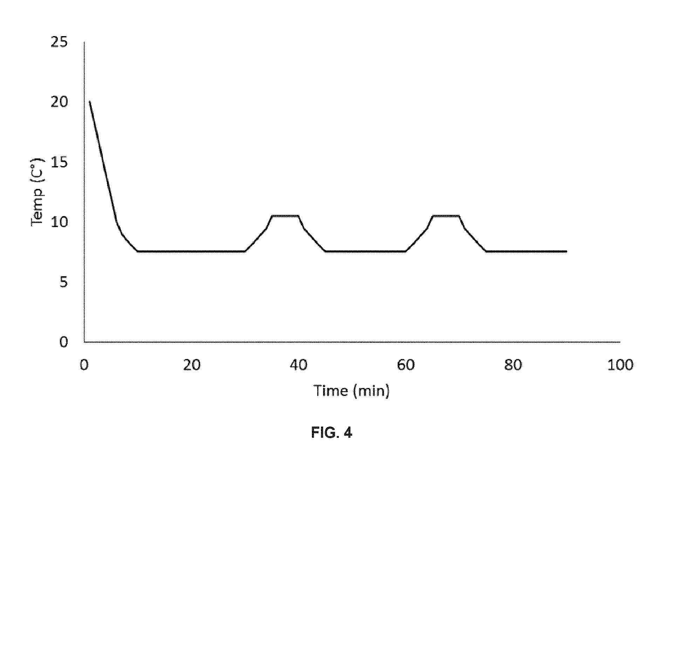

Figure 4 shows a reference treatment protocol for thermal treatment of a subacute superficial join injury.

DETAILED DESCRIPTION

The present invention will be described with respect to particular embodiments but the invention is not limited thereto but only by the claims. Any reference signs in the claims shall not be construed as limiting the scope thereof.
As used herein, the singular forms "a", "an", and "the" include both singular and plural referents unless the context clearly dictates otherwise.
The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" when referring to recited members, elements or method steps also include embodiments which "consist of" said recited members, elements or method steps.
Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.
The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.
The recitation of numerical ranges by endpoints includes all numbers and fractions subsumed within the respective ranges, as well as the recited endpoints.
All documents cited in the present specification are hereby incorporated by reference in their entirety.
Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.
Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.
In the State-of-the-Art, standard thermal treatment protocols are generally used in the field of therapeutic thermal care. These standard thermal treatment protocols aim to provide as-good-as possible thermal treatments for a given condition related to a given body part, for a large group of individuals. However, such standard thermal treatment protocols are oblivious to the fact that different individuals may have bodies with different thermal characteristics.

This can lead to the application of incorrect thermal treatments, thereby resulting in sub-par thermal care. The present invention provides a method, a computer program product, and a system which allows thermal treatment protocol individualization, thereby allowing for thermal treatment optimization, taking a specific individual's thermal characteristics into account.

In particular embodiments of the present invention, individual-specific information is used to create an individualized thermal model. This thermal model is then used to accurately predict the temperature distribution in tissues when a specific thermal treatment is applied. By comparing these predictions with known best practices in the field of therapeutic thermal care, an optimum thermal treatment protocol can be devised.

In a first aspect, the present invention provides a method for the personalized planning of the thermal treatment of an individual, the method comprising:

- providing a standard thermal treatment protocol adapted to the condition of said individual;
- receiving individual-specific information;
- applying said individual-specific information to said standard thermal treatment protocol, thereby adjusting parameters of said standard thermal treatment protocol such as the duration, number of intervals and/or intensity of the standard thermal treatment protocol; and
- outputting the adjusted thermal treatment protocol as the personalized protocol for the thermal treatment of said individual.

The use of personalized thermal treatment protocols feature significantly enhanced comfort levels and therapy effectiveness compared to standard thermal treatment protocols. The term "standard thermal treatment protocol" as used herein refers to known thermal treatment protocols which have been designed to be effective for an entire group of individuals. These are generic protocols based on literature references.

The term "personalized planning" as used herein refers to the process of selecting optimized thermal treatment protocols for an individual. The term "individual" as used herein refers to any person or animal undergoing a thermal treatment. An individual may be a patient, or an athlete. An individual may be an animal, for example a race horse. Preferably, the term "individual" refers to an athlete.

The term "thermal treatment" as used herein refers to the application of cryotherapy and/or thermotherapy as a therapeutic modality. Cryotherapy refers to the local or general use of low temperatures in the treatment of an injury, a medical condition, a disease or disorder. Generally, the application of cryotherapy results in the slow-down of an individual's metabolism. More specifically, cryotherapy aims to decrease cell growth and reproduction, increase cellular survival, decrease inflammation, decrease pain and spasm, promote the constriction of blood vessels, and when using extreme temperatures, to destroy cells by crystallizing the cytosol, which is the liquid found inside cells, also known as intracellular fluid. Typically, cold ice packs, cold pads, cold compresses, cold water and/or many others are used to perform cryotherapy. Thermotherapy refers to the local or whole-body use of high temperatures or heat in the treatment of a medical condition, a disease or disorder. Thermotherapy or heat therapy is most commonly used for rehabilitation purposes. The therapeutic effects of heat therapy include, a stimulation of an individual's metabolism, increasing the extensibility of collagen tissues; decreasing joint stiffness; reducing pain; relieving muscle spasms; reducing inflammation, oedema, aiding in the post-acute phase of healing; and increasing blood flow. The increased blood flow to the affected area provides proteins, nutrients, and oxygen for better healing. Typically, heating pads, hot compresses, hot-water bottles, hot water, ultrasound, hydro collator packs, whirlpool baths, cordless Far-infrared (FIR) heat therapy wraps, and/or many others are used to perform thermotherapy.

In particular embodiments, applying said individual-specific information to said standard thermal treatment protocol involves modelling heat transport within the individual or an anatomical part thereof and using the results of the heat transport modelling to adjust the parameters of said standard thermal treatment protocol. Typically, said modelling uses Pennes' general bioheat differential equation (for example in polar or spherical coordinates):

$$\rho c \frac{\delta T}{\delta t} = k \left( \frac{\delta^2 T}{\delta r^2} + \frac{\omega}{r} \frac{\delta T}{\delta r} \right) + q_m + c_{bl} \rho_{bl} w_{bl} (T_{bla} - T), \qquad (1)$$

in which $p$ is tissue density [$kg \cdot m^{-3}$], $c$ is tissue specific heat capacitance [$J \cdot kg^{-1} \cdot K^{-1}$], $T$ is tissue temperature [$K$], $t$ is time [$s$], k is tissue conductivity [$W \cdot m^{-1} \cdot K^{-1}$], r is a radial position coordinate [$m$], $\omega$ is a geometry factor ($\omega = 1$ in polar coordinates (2D); $\omega = 2$ in spherical coordinates (3D)), $q_m$ is the specific metabolic heat generation rate [$W \cdot m^{-3}$], $c_{bl}$ is blood's specific heat capacitance [$J \cdot kg^{-1} \cdot K{-1}$], $\rho_{bl}$ is blood density [$kg \cdot m^{-3}$], $w_{bl}$ is blood perfusion rate [$m^3 s^{-1} m^{-3}$], and $T_{bl}$ is arterial blood temperature [$K$]. The term "tissue" as used herein refers to the cellular organizational level intermediate between cells and a complete organ. Tissues typically used in modelling include the tissues present in the anatomical part of the individual that will undergo the thermal treatment. In particular, the skin layer, fat layer and muscle layer are included. Depending on the anatomy to be treated, also other anatomical tissues such as bones, or connective tissues such as fascia, tendons and/or ligaments may be included in the model.

Pennes' general bioheat differential equation provides an excellent description of many living tissues. Pennes' general bioheat equation can be solved when applying one or more appropriate boundary conditions to the system's boundaries. Boundary conditions are preferably related to manifestations of the physical characteristics of the boundaries of tissues under consideration, which are for example a fixed temperature at the tissue boundary, constant heat flow across the tissue boundary, resistive heat flow across the system boundary, heat flow continuity across the boundary between two different tissues, and/or temperature continuity across the boundary between two different tissues.

The process of solving Pennes' general bioheat equation subject to appropriate boundary conditions can be done by one or more methods chosen from the list comprising: the finite difference method, the method of lines, the finite element method, the finite volume method, spectral methods, and meshfree methods.

Preferably, thermoregulatory reactions of the central nervous system are taken into account. Such thermoregulatory reactions are for example suppression (vasoconstriction), elevation (dilatation) of the cutaneous blood flow; and shivering thermogenesis. When thermotherapy (the application of heat) is applied, elevation of the cutaneous blood flow is preferably taken into account. When cryotherapy (the application of cold) is applied, vasoconstriction of the cutaneous blood flow and shivering thermogenesis are preferably taken into account. These thermoregulatory reactions are preferably taken into account by modifying the specific metabolic heat generation rate, $q_m$, accordingly; and/or by modifying the boundary conditions accordingly. Taking these thermoregulatory reactions into account allows for the development of more effective thermal treatments.

Preferably, the application of said individual-specific information to said standard thermal treatment protocol according to the method of the invention, also involves the real-time measurement of individual-specific information, thereby taking into account one or more of the following parameters: skin temperature, core temperature, brain temperature, and changes in these temperatures (i.e. the partial derivatives of the respective temperatures with respect to time). Various physiological parameters can act as input for calculating individual-specific treatment protocols. For example, the following physiological parameters can be taken into account: heart rate, heart rate variability, respiration rate, neural oscillations, oxygen saturation, and subjective perceived comfort. Measuring these parameters, and taking them into account as appropriate parameters or boundary conditions for thermal tissue simulations allows for more accurate modelling, and for the development of more accurate thermal treatments.

Preferably, the real-time measurement of individual-specific information involves the application of one or more thermal pads (heating and/or cooling pads) on one or more body parts to be treated. The thermal pads may be efficient means of providing heat to a treated body part. In particular embodiments, these thermal pads comprise sensors for measuring individual-specific information.

Apart from parameters such as skin temperature, head core temperature, and skin temperature change, the individual-specific information may also be the thermal resistance between the thermal pad and the skin. A significant thermal resistance between thermal pad and skin may result in a significant temperature difference between thermal pad and skin; this temperature difference is preferably taken into account in order to achieve a more accurate prediction of the temperature in the tissue to be treated, and therefore allowing to device a more effective treatment.

The term "thermal resistance" as used herein refers to a thermal material property which describes the extent to which an object, tissue, material, fluid, device or boundary layer hinders the flow of heat when subjected to a temperature gradient. In particular, the thermal resistance is defined as the temperature difference across a structure when a unit of heat energy flows through that structure in one time unit. The SI units of thermal resistance are Kelvin per Watt.

In particular embodiments, the individual-specific information involves taking into account the thermo physical properties of the clothing worn by the individuals, for example the clothing's thermal resistance. Many types of clothing significantly affect the exchange of heat between an individual and the environment. As a result, their presence is preferably taken into account when applying said individual-specific information to said standard thermal treatment protocol. This is particularly relevant when whole-body thermal treatments are performed according to methods of the present invention; for example in cryotherapy chambers, heated chambers or water baths.

In particular embodiments, the heat transport modelling involves dynamically modelling an individual's response to an applied thermal treatment. For example, this can be done using a method which comprises three steps: (i) modelling the human body's thermal characteristics, (ii) modelling heat-transport mechanisms within the body and at its periphery and (iii) numerically solving the resulting equations with appropriate boundary conditions. Doing so may result in achieving more realistic modelling results, thereby creating the opportunity to achieve more optimized thermal treatments. Modelling the human body's thermal characteristics can be done using D. Fiala's model [Fiala D, Lomas KJ, Strohrer M (1999) A computer model of human thermoregulation for a wide range of environmental conditions: the passive system, Journal of Applied Physiology, pp. 1957-1972], which is hereby incorporated by reference in its entirety. This is an efficient model of an individual's thermal characteristics.

Preferably, different standard treatment temperatures are applied to different joints and/or muscles; and treatment temperature individualization is performed according to different protocols, depending on the joint and/or muscle under treatment. The term "standard treatment temperature" as used herein refers to the treatment temperature of the standard thermal treatment protocols.

Preferably, the treatment temperature for a given joint or muscle is adapted according to the specific characteristics of that joint or muscle and according to the individual-specific information.

For example, for personalized treatment protocols for cryotherapy using pads, knees, shoulders and hips can be treated using personalized cryotherapy protocols featuring a standard temperature between 6°C and 10°C, preferably about 8°C; lower leg muscles and upper arm muscles can be treated with personalized protocols featuring a standard temperature between 8°C and 12°C, preferably about 10°C; elbows, ankles, and forearm muscles can be treated using personalized protocols featuring a standard temperature between 10°C and 14°C, preferably about 12°C; wrists can be treated using personalized protocols featuring a standard temperature between 11°C and 15°C, preferably about 13°C.

Different joints and muscles have their own unique properties. Therefore, treatment protocols are preferably adapted to take these unique properties into account. Preferably, the temperature modulation range is adapted depending on the joint or muscle being treated and according to the characteristics of the treated individual's body. The term "temperature modulation range" as used herein refers to the range in which the treatment temperature varies during the thermal treatment of an individual. The temperature modulation range is preferably chosen to optimize an individual's comfort level without compromising treatment efficacy.

The temperature of the treatment protocol is preferably also modulated. Subjecting body parts to prolonged periods at constant low temperatures typically causes discomfort. Therefore, during the treatment protocol small modulations of the temperature may be incorporated in the protocol. These modulations are short periods where the temperature is increased or decreased with 0.5°C to 5°C, thereby alleviating the discomfort for the individual.

It should be noted that the specific temperatures which are used also depend on the cryotherapy or thermotherapy methods which are used. For example, cryotherapy treatment protocols based on water immersion generally require less cool fluid temperatures compared to cryotherapy treatment protocols based on cold-pad cooling because heat transfer is much more efficient in the former method. This is related to the fact that the water-skin boundary's thermal resistance is much lower than the cold pad-(air)-skin boundary layer's thermal resistance. For a similar reason, thermotherapy using water immersion requires the application of cooler temperatures compared to thermotherapy based on heat pads.

As different joints and muscles have their own unique properties, temperature modulation ranges are preferably adapted to take these unique properties into account.

In particular embodiments, the individual-specific information, as used in methods according to the invention, comprises general information chosen from age, gender, mass, height, fitness or a combination thereof as well as individual-specific anatomical information such as subcutaneous fat content of the treated body part, the type of tissue (muscle, tendon, joint), the depth of the injury or a combination thereof.

The inventors have come to the remarkable discovery that the use of such parameters which relate to an individual's overall characteristics can be used to accurately predict the local properties of specific tissues such as joints or muscles; thereby allowing highly efficient individualized thermal treatments. For example, the individual-specific information comprises an individual's age, gender, mass, height, fitness, subcutaneous fat content of the treated body part, size or circumference of joint, and the depth of the injury. The term "general information" as used herein refers to an individual's overall characteristics. Preferably, these include an individual's gender, age, body height, body weight, stature, muscle mass, and/or training status.

The term "individual-specific information" as used herein refers to information relating to a single individual. The term "fitness" as used herein refers to a measure for a person's state of health, usually as a result of exercise and nutrition. The term "subcutaneous fat content (SFC)" as used herein refers to the mass of the panniculus adiposus, relative to the whole body mass. The panniculus adiposus is the fatty layer of the subcutaneous tissues, it is superficial to a deeper layer of muscle tissue, the panniculus carnosus. The term "treated body part" as used herein refers to the part of an individual's body which is undergoing thermal treatment. The term "depth of injury" as used herein refers to the smallest distance between the position of an injury and the outside of an injured individual's body.

Preferably, as much as possible individual specific information is taken into account, subject to the condition of relevancy. Individual specific information is considered to be relevant when it can be reasonably expected to influence an individual's thermal characteristics.

In particular embodiments, the subcutaneous fat content of the treated body part is calculated based on the individual-specific information. In particular, the subcutaneous fat content (SFC) may be calculated from the whole body fat content (BF), the visceral adipose tissue content (VAT), the abdominal subcutaneous adipose tissue content (ASAT), and the actual body mass (mass), according to the following equation:

$$SFC = \frac{BF - VAT - ASAT}{mass}. \qquad (2)$$

This appears to yield a remarkably accurate determination of the SFC.

The term "actual body mass (mass)" as used herein refers to the total mass of an individual's body. This parameter is typically measured using the typical methods known in the art. The term "abdominal subcutaneous adipose tissue content (ASAT)" as used herein refers to the mass of an individual's abdominal subcutaneous adipose tissue. The term "whole body fat content (BF)" as used herein refers to the total mass of an individual's fat tissues. The term "visceral adipose tissue content (VAT)" as used herein refers to the mass of an individual's visceral adipose tissue, i.e. fat tissue around internal organs.

Alternatively, the SFC is measured, for example using body fat callipers. The term "Body fat callipers" as used herein refers to one or more devices configured to allow for measurements of the distance between two opposite sides of an object.

Preferably, a maximum SFC value, $SFC_{max}$, is defined; for example, $SFC_{max} = 0.11$. Such an upper limit is useful under certain conditions when computed SFC values could be unrealistic. The application of an upper limit therefore improves the method's robustness.

The whole body fat content ($BF$) may be obtained from the individual's body mass index ($BMI$), age ($age$), and the individual's status according to the following equation:

$$BF = c_{bf,b} \cdot BMI + c_{bf,a} \cdot age + c_{bf,0} + c_{bf,s} \cdot status, \qquad (3)$$

in which $c_{bf,b}$, $c_{bf,a}$, $c_{bf,0}$, and $c_{bf,s}$ are coefficients which depend on the individual's sex. This is an efficient and pragmatic method to determine the whole body fat content.

The term "body mass index (BMI)" as used herein refers to a measure of relative weight based on an individual's mass and height; the BMI equals an individual's mass divided by the square of the individual's length, in units of $kg/m^2$.

The term "status" as used herein refers to a numerical indicator for the training status of a person. More specifically, the term "status" as used herein refers to an indicator for the person's muscle mass content. The status can vary between 0 (untrained) and 1 (trained) depending on the training status and the sports discipline of the athlete. For example, the status variable is different for soccer players, for rugby players, and for weightlifters.

Preferably, the whole body fat content (BF) is treated as a user-defined parameter. This can be useful when the whole body fat content has been measured.

The visceral adipose tissue (VAT) may be determined from [Han TS, Lean MEJ (2001) Anthropometric Indices of Obesity and Regional Distribution of Fat Depots. International Textbook of Obesity. Ed. Per Bjorntorp, John Wiley & Sons Ltd, pp. 51-65]:

$$VAT = BF\left(c_{vat,bf0} + c_{vat,bfa} \cdot age\right) + c_{vat,aa} \cdot age^2 + c_{vat,a} \cdot age + c_{vat,0}, \qquad (4)$$

in which $c_{vat,bv0}$, $c_{vat,bva}$, $c_{vat,aa}$, $c_{vat,a}$, and $c_{vat,0}$ are gender-specific coefficients. When used in conjunction with adequate numerical values for these coefficients, this equation yields a remarkably accurate value for the visceral adipose tissue.

The abdominal subcutaneous fat content (ASAT) may be computed from experimental results according to [Han TS, Lean MEJ (2001) Anthropometric Indices of Obesity and Regional Distribution of Fat Depots. International Textbook of Obesity. Ed. Per Bjorntorp, John Wiley & Sons Ltd, pp. 51-65. 43], [Kuk JL, Lee SJ, Heymsfield SB, Ross R (2005) Waist circumference and abdominal adipose tissue distribution: influence of age and sex. Am J Clin Nutr 81: 1330-1334], [Weiss WW, Clark FC (1987) Three protocols for measuring subcutaneous fat thickness on the upper extremities. European Journal of Applied Physiology and Occupational Physiology 56 (2) 217-221]:

$$ASAT = c_{as,b} \cdot BF + c_{as,a} \cdot age + c_{as,0}, \qquad (5)$$

in which $c_{as,b}$ and $c_{as,a}$ are gender-specific coefficients. This is a pragmatic and efficient way to calculate the ASAT.

The SFC, BF, VAT, and/or ASAT may be used to compute the local adipose and fat-free mass portions by means of an iterative procedure.

As the local adipose and fat-free mass portions determine the individual's local thermal properties, this can be a very useful step in determining an individual's optimal thermal treatment parameters.

Also, an iterative procedure may distribute the adipose tissue by scaling local subcutaneous fat layers based on relative proportions of the subcutaneous fat tissue mass in body elements defined in a reference model. This allows for accurate modelling of an individual's local thermal properties. The term "reference model" as used herein refers to a 'standard human' which reproduces the average observed characteristics from anthropometric studies including for example the dimensions (and mass) of individual body parts, the total body height, mass, fat content, average body density, body surface area, basal metabolic and basal cardiac output rates.

Preferably, the *age* parameter is a modified age ($age_m$). For example, $age_m$ may be calculated according to a specific equation taking into account the difference between an individual's actual age and a reference age corresponding to a reference person. For example, for a reference age of 27 years, an age deviation $\Delta N_{age}$ can be defined as:

$$\Delta N_{age} = sign(age_r - 27) \cdot \left[ 2 \frac{(age_r - 27)^2 - 25}{(age_r - 27)^2 + 25} + 2 \right], \tag{6}$$

in which $age_r$ is an individual's actual age, the *sign* function yields the sign of the expression between brackets, and $\Delta N_{age}$ is used to calculate the *age* parameter according to:

$$age_m = age_r + \Delta N_{age}. \tag{7}$$

The use of a modified age parameter, $age_m$, instead of an individual's actual age, $age_r$, can yield a more accurate prediction of an individual's (local) tissue properties.

Preferably, algorithms used as part of the present invention are implemented to enable user-specified adjustments. Therefore, the models can be adapted according to new emerging insights.

In particular embodiments, applying said individual-specific information to said standard thermal treatment protocol, as used in the method according to the invention, involves the use of personalized simulations taking into account one or more of the following pieces of information: the individual's gender, age, body height stature and training status. The inventors have remarkably discovered that taking these factors into account allows for a more accurate determination of an individual's local thermal properties.

The inventors have surprisingly found that thermal treatment individualization can be performed using one or more overall body characteristics. This is quite remarkable since the local heat transfer processes (and thus deep tissue temperatures) would seem to be primarily affected by local characteristics. The inventors discovered that local properties can be accurately modelled from an individual's overall characteristics to a degree in which an individual's overall characteristics can be used to predict local body properties.

In particular embodiments, the individual-specific information, as used in the method according to the invention, is obtained from anatomy measurements of the individual.

Using this information as input for treatment personalization protocols allows for efficient protocol individualization.

The term "anatomy measurements" as used herein refers to the measurement of certain characteristics of an individual. Anatomy measurements can be very easy and fast measurements yielding very general information, such as length measurements. For example, anatomy measurements may comprise weight measurements. For example, anatomy measurements may comprise arm length measurements. For example, anatomy measurements may comprise thorax circumference measurements. For example, anatomy measurements may comprise leg length measurements.

For example, anatomy measurements may feature the use of one or more medical imaging techniques chosen from the list comprising: radiotherapy, magnetic resonance imaging (MRI), ultrasound, elastography, tactile imaging, photoacoustic imaging, thermography, tomography, and echocardiography.

In particular embodiments, the standard thermal treatment protocol, as used in the method according to the invention, is a generic thermal treatment protocol function of medical knowledge collected from literature converted into a functional thermal treatment protocol, preferably based on for instance the anatomical region to be treated, the structure, the recovery phase, the depth of the injury, the level on severity of the injury or a combination thereof.

The term "structure" as used herein refers to the anatomical part of the body that is undergoing thermal treatment. More specifically, the term "structure" refers to the distinction between muscles, tendons, and joints.

Injuries can generally be divided in different categories or levels. A level 1 injury is the least severe and refers to a strain. A level 2 injury is an intermediate level which involves partial or complete muscle or tendon rupture. A level 3 injury refers to the most severe type of injury which involves the features of level 2 injuries (partial or complete muscle or tendon rupture) plus additional complications, especially avulsion. Major crush injuries may also be categorised as level three injuries. In literature also other classification systems have been described.

Defining treatment protocols in this way, and modifying the functional treatment protocol based on the above-mentioned parameters allows for a highly effective treatment protocol individualization.

The term "functional thermal treatment protocol" refers to a thermal treatment protocol which has been adapted to the treatment of a specific body part, of a specific individual, for a specific medical condition.

In particular embodiments, the present invention provides that applying said individual-specific information to said standard thermal treatment protocol results in an adaptation of the temperature parameters, duration of the temperature intervals, frequency of the temperature intervals, the frequency of the cycles of thermal treatment, the slope of temperature changes, the overall duration of the protocol temperature intervals or a combination thereof.

Allowing the adaptation of these thermal treatment protocol parameters yields a remarkable amount of degrees of freedom for thermal treatment individualization. The term "temperature parameters" as used herein refers one or more physical parameters which taken together in the appropriate context, define the thermal treatment. The term "temperature intervals" as used herein refers to a time of elevated or depressed temperature in a given period (100). The term "frequency of the temperature intervals" as used herein refers to the inverse of the period (100) in which a sequence of one or more temperature intervals is repeated. The term "thermal treatment cycle" as used herein refers to the temperature profile within one period (100). The term "frequency of the cycles of thermal treatment" as used herein refers to the inverse of the time scale in which a sequence of one or more thermal treatment cycles is repeated. It is the inverse of a period. The term "slope of temperature changes" as used herein refers to the derivative of the treatment temperature with respect to time. The term "overall duration of the protocol temperature intervals" as used herein refers to the total duration of a thermal treatment.

Preferably, the frequency with which thermal treatment protocols are applied is personalized according to an individual's characteristics.

In particular embodiments, the adjusted thermal treatment protocol is evaluated prior to outputting the adjusted thermal treatment protocol to monitor whether the adjusted parameters do not exceed pre-set thresholds. These pre-set thresholds are particularly included for safety-reasons and for physical comfort considerations.

Some thermal treatment individualization protocols may be inaccurate when applied to individuals who differ very strongly from the reference individual. The definition of pre-set thresholds for thermal treatment parameters allows for the application of more optimal thermal treatment protocols in these cases.

In particular embodiments, the thermal treatment protocol, as used in the method according to the invention, comprises an inslope phase, a treatment phase, and an outslope phase.

A relationship exists between heat and cold receptors in human skin and their discharge rate which can cause a sensation of pain when a cooling or heating rate is too steep. The application of inslope and outslope phases allows for gradual temperature changes, which improve the treated individual's comfort level.

The term "inslope phase" as used herein refers to a pre-treatment phase in which the temperature applied to an individual is changed gradually from a standby temperature to a treatment temperature. Preferably, the "inslope phase" is individualized according to an individual's bodily characteristics.

The term "outslope phase" as used herein refers to a post-treatment phase in which the temperature applied to an individual is changed gradually from a treatment temperature to a standby temperature. Preferably, the "outslope phase" is individualized according to an individual's bodily characteristics.

Preferably, the inslope phase is designed to avoid tissue damage or excessive defence mechanisms in human tissue. Preferably, the outslope phase at the end of a thermal treatment protocol is designed to minimize the rebound effect that takes place in human tissue after the physiological application of cold. In addition, the outslope may facilitate rehabilitation exercises after the application of a thermal treatment protocol to an individual.

The term "treatment temperature" as used herein refers to the average applied temperature during a thermal treatment. The term "standby temperature" as used herein refers to the temperature at which a thermal treatment begins and/or ends.

Depending on the characteristics of a treated individual, the pre- and post-treatment standby temperatures may differ, or they may be the same. This additional degree of freedom allows for further thermal treatment optimization, thereby providing the opportunity for more optimized thermal treatment design.

In particular embodiments, the personalized thermal treatment protocol, as provided in the method according to the invention, is executed on the individual once the personalized thermal treatment protocol has been determined for the individual.

In particular embodiments, the method according to the present invention provides that temperature data information is received from temperature sensors on the skin of said individual prior to or during the personalized thermal treatment of the individual and applying said temperature data information to the personalized thermal treatment protocol, thereby further adjusting said personalized thermal treatment protocol. Also, other parameters may be taken into account such as the perceived comfort, muscle tension, breathing rate, blood velocity, blood pressure, and neural oscillations. In general, any type of biofeedback (i.e. responses of the body with respect to applied thermal treatments) can be taken into account. Such real-time measurements can provide useful information for further thermal treatment optimization.

Effective thermal treatment entails exchanging an amount of energy (heat or cold) with the body during a certain span of time and following a particular treatment sequence of treatment cycles. The optimal amount of energy exchanged depends on many factors that are difficult to control and which vary from patient to patient and from injury to injury. Skin temperature sensing allows for an accurate control of the heat provided to, or extracted from, a thermally treated body part. Therefore, there is a need for temperature sensing and a feedback mechanism to ensure an optimized thermal treatment.

In particular embodiments, thermal treatment optimization makes use of skin conductivity measurements, skin colour measurements, skin temperature measurements, skin texture measurements, heart rate measurements, and/or blood saturation measurements.

In a second aspect, the present invention provides a computer program product comprising one or more computer readable media having computer executable instructions for performing the steps of the method according to the first aspect of the present invention or of any particular embodiment thereof.

In some cases, computer implementations of the present invention can be particularly effective.

Preferably, the computer program product further comprises a storage means adapted to store the acquired data and to document the adapting of the thermal treatment.

For example, the documentation of the thermal treatment may be realized by generating a protocol and particularly safe/store the protocol on a processor unit which may be part of the thermal treatment device. A protocol may contain data and/or commands and/or sequences of commands and may be adapted to control the operation of the thermal treatment.

Preferably, the computer program product comprises a system for generating a feedback-driven protocol for thermal treatment of an individual's body part.

In particular embodiments, an individual's temperature distribution data may be obtained by an IR (infrared) camera and may be displayed to the physician and/or used for feedback control of the thermal treatment.

Preferably, the time lapsed during the thermal treatment is taken into account when controlling the thermal treatment based on the determination of various body part related parameters.

In a third aspect, the present invention provides a system for the automated planning of the thermal treatment of an individual or a part thereof with individual-specific consideration, the system comprising

- a thermal treatment protocol database which stores one or more standard thermal treatment protocols; said standard thermal treatment protocols being generic thermal treatment protocols in function of medical knowledge collected from literature converted into a thermal treatment protocol, preferably based on for instance the anatomical region to be treated, the structure, the recovery phase, the depth of the injury, the level on severity of the injury or a combination thereof;
- one or more processors programmed to receive a plurality of individual-specific parameters of said individual or a part thereof; and information about the required thermal treatment for said individual or a part thereof;

said one or more processors being operable to apply to a standard thermal treatment protocol the individual-specific parameters thereby adjusting parameters of said standard thermal treatment protocol such as the duration, number of intervals and/or intensity of the standard thermal treatment protocol and generating an adjusted thermal treatment protocol as the personalized protocol for the thermal treatment of said individual or a part thereof; and; said one or more processors being operable to display the personalized protocol for the thermal treatment of said individual.

This system allows for an efficient and semi-automated determination and application of personalized thermal treatment protocols.

The system may have a printer device which can either be embedded or external, and may be configured to communicate wirelessly to the processors.

In particular embodiments, the system according to the invention comprises an input operable to receive said individual-specific parameters of said individual; and the information about the required thermal treatment for said individual. The input device facilitates communication between a human and the system.

Preferably, the input device is an interactive interface which is configured to provide direct feedback to a user. For example, the interface may be a touch screen, but other user interfaces may be used as well, for example a hardware keyboard, a pointing device, and/or audio input.

In particular embodiments, the system according to the invention comprises measurement means adapted to acquire data of a thermal treatment from the individual; and means for adapting the thermal treatment protocol of the individual on the basis of the data acquired.

Preferably, the thermal treatment protocol is adapted based on a comparison of measured data with predetermined data in a data storage unit.

Such real-time feedback can allow real-time thermal treatment optimization. In addition, the gathered information can be used for the further optimization of thermal treatment personalization protocols.

For example, the amount of thermal exchange between a thermal pad and the skin can be determined by using a temperature sensitive surface between the thermal pad and the skin. Alternatively, a measurement of the power output to the fluid in the thermal pad itself can provide an indirect determination of the amount of thermal exchange with a body.

Alternatively, a direct measurement of biofeedback, e.g. treated body part temperature, from a patient can be obtained from a sensor to obtain a reading from a e.g. drainage catheter, which, for example, has been inserted into the body following knee reconstructive surgery;

In particular embodiments, a system according to the present invention comprises one or more thermal modalities chosen from the list consisting of: hydraulic devices, Peltier thermal exchange elements, water immersion facilities, bath tubs, and cryogenic chambers. Peltier elements can be very efficient thermal exchange elements because they are solid state devices and do not require recirculated fluids, which thermal treatment system design. However, hydraulic devices are generally more energy efficient compared to Peltier elements. Water immersion facilities have the advantage of being especially effective in applying whole-body thermal treatment protocols. Bath tubs are a preferred choice when most of the body should be treated, except for the head. Also, ergonomically shaped bath tubs allow for excellent comfort of use during thermotherapy, facilitating the application of extended treatments. Cryogenic chambers are effective facilities for applying very cold temperatures to an individual for a short time.

In particular embodiments, the system comprises infrared radiators configured to heat a subject's body parts under treatment. Infrared radiators allow superficially heating a body part from a distance, i.e. without the need for close contact. This may be useful when, for example, touching the treated body part with a heat pad would cause significant pain.

The term "heat pad" as used herein refers to a pad used for warming parts of the body, for example to manage pain. The local application of heat causes blood vessels in the treated area to dilate, thereby enhancing blood perfusion to the treated tissue. In some embodiments, a heat pad may comprise the term "cold pad". The term "cold pad" as used herein refers to a pad used for cooling parts of the body, for example to manage pain.

Preferably, a heat pad is a disposable unit which is replaced after a pre-determined amount of time, due to treatment protocols, safety, or expiry of the pad. For example, the heat pad can be disabled after ten hours of treatment.

The system may comprise an ultrasound diathermy module in which high-frequency acoustic vibrations are used which, when propelled through the treated tissues, are converted into heat. The term "ultrasound diathermy" as used herein refers to a physical therapy in which comprises the use of alternating compression and rarefaction of sound waves with a frequency of >20,000 cycles/second. Typically, frequencies of 0.7 to 3.3 MHz are used.

The term "High-frequency acoustic vibrations" refer to acoustic vibrations with frequencies commonly used in ultrasound diathermy, i.e. >20,000 cycles/second; typically, frequencies of 0.7 to 3.3 MHz.

Ultrasound diathermy is suited for speeding up of the healing process from the increase in blood flow in the treated area. Ultrasound diathermy is also suited for decreasing pain by reducing swelling and oedema. Also, ultrasound diathermy results in the massage of muscles, tendons and/ or ligaments in the treated area. The beneficial effects of sound wave diathermy are related to both thermal and non-thermal effects. Thermal effects are due to sound wave absorption. Non-thermal effects are from e.g. cavitation and acoustic streaming. Ultrasound diathermy can be used for example for the treatment of ligament sprains, muscle strains, or impingement syndrome.

In particular embodiments, the system comprises a short wave diathermy module. The term "Short wave diathermy module" as used herein refers to a device for performing short wave diathermy operations which use electromagnetic radiation in ISM band frequencies, typically frequencies of 13.56, 27.12, and/or 40.68 megahertz.

Short wave diathermy is specifically suited for the treatment of deep muscles and joints that are covered with a heavy soft-tissue mass, for example the hip. In some instances short wave diathermy may be applied to localize deep inflammatory processes, as in pelvic inflammatory disease.

In particular embodiments, the system comprises an infrared diathermy module, which can be specifically effective for relieving for example minor muscle or joint stiffness, minor muscle strains and/or spasms. Infrared diathermy may also result in a temporary increase in local circulation, and in muscle relaxation where applied. The term "infrared diathermy module" as used herein refers to a device for performing infrared diathermy operations, which is configured to emit electromagnetic radiation in the infrared spectrum to provide local heating for the purpose of temporarily the local tissue temperature.

In particular embodiments, the system comprises a microwave diathermy module.

The term "microwave diathermy module" as used herein refers to a device for performing infrared diathermy operations in which microwave radiation is used for heat generation. Microwave diathermy is a particularly easy-to-use way to apply heat to a treated area.

In particular embodiments, the present invention may be applied in the field of cranial cooling, for example for reducing neurological deterioration in trauma victims by introducing a hypothermic state. For example, a method according to the present invention can be applied in conjunction with the headwear reactor disclosed in US patent no. 2010/0319110, which is hereby incorporated in its entirety. Cranial cooling has been noted to reduce brain damage and increase survival rates in accident victims, and patients with head injuries are often treated in accident and emergency centres. However, the shape and size of the heads of different individuals is generally different. Consequently, there exists significant variation in head properties in a population. Not taking these variations into account may lead to sub-par thermal treatments, i.e. it may lead to too much or too little cooling. Too little cooling may render a cranial cooling treatment ineffective whereas too much cooling may lead to excessive hypothermia and related injuries. Methods according to the present invention allow taking the thermal characteristics of an individual's head into account, thereby improving the efficacy of cranial cooling and reducing the chance of side effects.

In a particular embodiment, the thermal treatment of an individual, as defined in the methods and systems described herein, is combined with other types of treatments (referred to as additional types of treatments), including for instance compression or intermittent compression, vibration, electric stimulation or ultrasound. Also a combination of the thermal treatments with the use of active ingredients is envisaged. In particular embodiments, the individual-specific information or part thereof is used to adjust or determine the parameters of the additional types of treatments. In particular, the individual-specific information or part thereof is used to adjust or determine the parameters of the compression or intermittent compression treatment.

**EXAMPLES.**

**Example 1:**

The first example provides a particular embodiment of the present invention in which a standard thermal treatment protocol is provided. The standard thermal treatment protocol is adapted to the characteristics of an individual undergoing thermal treatment. The person's BMI, BF, VAT, ASAT, length, and weight are determined according to particular embodiments of the present invention. Using inter alia this information, the parameters of the Fiala model of human thermoregulation are determined. This model is used to predict changes in tissue temperature when the tissue under consideration is subjected to a thermal treatment. By comparing these predictions with medical knowledge in the field of therapeutic thermal care, an optimal personalized temperature profile is selected for a specific individual having a specific medical condition in a specific body part. Using the individual's personal characteristics allowed to predict the optimal thermal treatment protocol for that specific individual, thereby providing more efficient therapeutic thermal care compared to standard treatment protocols.

**Example 2:**

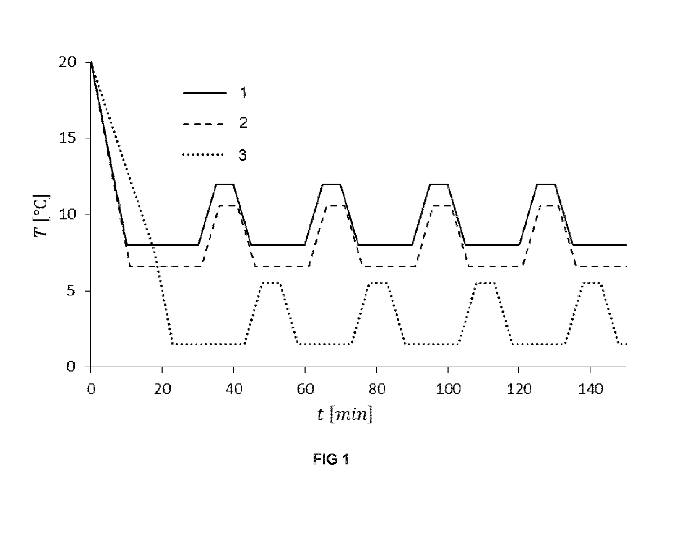

The second example provides a particular embodiment of the present invention which comprises the thermal treatments shown in Figure 1. Figure 1 shows three different thermal treatment protocols for an acute, level 2, superficial upper leg muscle injury.

A limb injury is described as 'superficial' when the strain involved in the injury penetrates to a depth shallower than 50% of the injured limb's radius. Superficial limb injuries should be contrasted with deep limb injuries. In deep limb injuries, the strain penetrates deeper than 50% of the radius of the injured limb.

Protocol (1) is a reference treatment protocol, protocol (2) is a treatment protocol for a first individual, and protocol (3) is a treatment protocol for a third individual. Personalized protocols (2) and (3) are derived from the standard treatment protocol, taking the characteristics of respectively the first and second individuals into account. In the present example, the average treatment temperature and the duration of temperature fluctuations were varied based on the individual characteristics, keeping other parameters constant.

The individual-specific information taken into account for individualizing the treatment protocol for individual 1 comprises: he is a 28 year old male soccer player with a height of 190 cm, weighing 94 kg, the length of his legs is 100 cm. The individual-specific information taken into account for treatment protocol individualization for individual 2 comprises: he is a 22 year old male soccer player with a height of 199 cm, weighing 91 kg, the length of his legs is 107 cm.

**Example 3**

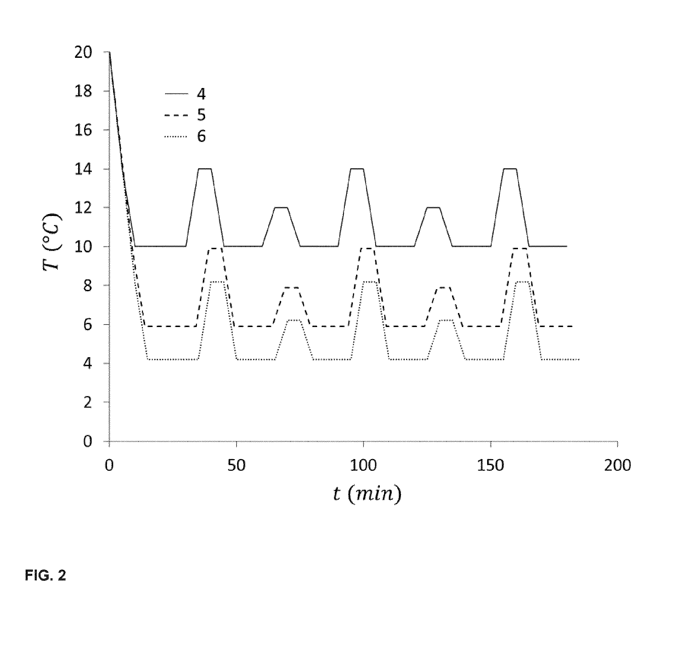

The third example provides a particular embodiment of the present invention which comprises the thermal treatments shown in Figure 2. Figure 2 shows three different thermal treatment protocols for an acute, level 2, deep, lower leg muscle injury. Protocol (4) is a reference treatment protocol, protocol (5) is a treatment protocol for a first individual, and protocol (6) is a treatment protocol for a third individual. Personalized protocols (5) and (6) are derived from the standard treatment protocol, taking the characteristics of respectively the first and second individuals into account. In the present example, the average treatment temperature and the duration of temperature fluctuations were varied based on the individual characteristics, keeping other parameters constant.

The individual-specific information taken into account for individualizing the treatment protocol for individual 1 comprises: he is a 23 year old male soccer player with a height of 170 cm, weighing 74 kg, the length of his legs is 88.5 cm. The individual-specific information taken into account for individual 2 comprises: he is a 21 year old male soccer player with a height of 193 cm, weighing 95 kg, the length of his legs is 103 cm.

**Claims**

1. Method for the personalized planning of the thermal treatment of an individual, the method comprising:

   - providing a standard thermal treatment protocol adapted to the condition of said individual;
   - receiving individual-specific information;
   - applying said individual-specific information to said standard thermal treatment protocol, thereby adjusting parameters of said standard thermal treatment protocol such as the duration, number of intervals and/or intensity of the standard thermal treatment protocol; and
   - outputting the adjusted thermal treatment protocol as the personalized protocol for the thermal treatment of said individual.

2. The method according to claim 1, wherein said individual-specific information comprises general information chosen from age, gender, mass, height, fitness or a combination thereof as well as individual-specific anatomical information such as subcutaneous fat content of the treated body part, the depth of the injury or a combination thereof.

3. The method according to claim 2, wherein the subcutaneous fat content of the treated body part is calculated based on the general information.

4. The method according to any of claims 1 to 2, wherein the individual-specific information is obtained from anatomy measurements of the individual.

5. The method according to any of claims 1 to 4, wherein said standard thermal treatment protocol is a generic thermal treatment protocol function of medical knowledge collected from literature converted into a functional thermal treatment protocol, preferably based on for instance the anatomical region to be treated, the structure, the recovery phase, the depth of the injury, the level on severity of the injury or a combination thereof.

6. The method according to any of claims 1 to 5, wherein applying said individual-specific information to said standard thermal treatment protocol results in an adaptation of the temperature parameters, duration of the temperature intervals, frequency of the temperature intervals, the frequency of the cycles of thermal treatment, the slope of temperature changes, the overall duration of the protocol temperature intervals or a combination thereof.

7. The method according to any of claims 1 to 6, wherein prior to outputting the adjusted thermal treatment protocol, the adjusted thermal treatment protocol is evaluated to monitor whether the adjusted parameters do not exceed pre-set thresholds.

8. The method according to any of claims 1 to 7, wherein said thermal treatment protocol comprises an inslope phase, a treatment phase and an outslope phase.

9. The method according to any of claims 1 to 8, wherein once the personalized thermal treatment protocol has been determined for the individual, the personalized thermal treatment protocol is executed on the individual.

10. The method according to any of claims 1 to 9, wherein said method further comprises receiving information from sensors on the skin of said individual during the personalized thermal treatment of the individual and applying said information to the personalized thermal treatment protocol, thereby further adjusting said personalized thermal treatment protocol.

11. Computer program product comprising one or more computer readable media having computer executable instructions for performing the steps of the method according to any of claims 1 to 10.

12. A system for the automated planning of the thermal treatment of an individual or a part thereof with individual-specific consideration, the system comprising

   - a thermal treatment protocol database which stores one or more standard thermal treatment protocols; said standard thermal treatment protocols being generic thermal treatment protocols in function of medical knowledge collected from literature converted into a thermal treatment protocol, preferably based on for instance the anatomical region to be treated, the structure, the recovery phase, the depth of the injury, the level on severity of the injury or a combination thereof;

- one or more processors programmed to receive a plurality of individual-specific parameters of said individual or a part thereof; and information about the required thermal treatment for said individual or a part thereof;

said one or more processors being operable to apply to a standard thermal treatment protocol the individual-specific parameters thereby adjusting parameters of said standard thermal treatment protocol such as the duration, number of intervals and/or intensity of the standard thermal treatment protocol and generating an adjusted thermal treatment protocol as the personalized protocol for the thermal treatment of said individual or a part thereof; and;
said one or more processors being operable to display the personalized protocol for the thermal treatment of said individual.

13. System according to claim 12, further comprising an input operable to receive said individual-specific parameters of said individual; and the information about the required thermal treatment for said individual.

14. System according to claim 12 or 13, further comprising measurement means adapted to acquire data of a thermal treatment from the individual; and means for adapting the thermal treatment protocol of the individual on the basis of the data acquired.

15. System according to any of claims 12 to 14, further comprising one or more thermal modalities chosen from the list consisting of: hydraulic devices, Peltier thermal exchange elements, water immersion facilities, bath tubs, and cryogenic chambers.

FIG 1

FIG. 2

FIG. 3

FIG. 4

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Jozef Stefan Institute: "Smart thermal therapy", , 18 February 2014 (2014-02-18), page 1000, XP055196213, Retrieved from the Internet: URL:http://www.ip4smes.eu/attachment/coope ration_profile_SmartThermalTherapy.pdf [retrieved on 2015-06-16] * abstract; figures 1,2; table 1 * * page 1, column 1, paragraph 1 - page 2, column 1, paragraph 5 * | 1-15 | INV. A61F7/00 G06F19/00 |
| A | Dusan Fiala ET AL: "A computer model of human thermoregulation for a wide range of environmental conditions: the passive system", Journal of Applied Physiology, 1 January 1999 (1999-01-01), XP055196398, Retrieved from the Internet: URL:http://jap.physiology.org/content/jap/ 87/5/1957.full.pdf [retrieved on 2015-06-17] * abstract * * page 1957, column 2, paragraph 4 - page 1958, column 1, paragraph 2 * * page 1961, column 1, paragraph 2 * | 1-15 | |
| A | Healthline Editorial Team: "Treating Pain & Injury With Heat and Cold", , 9 July 2014 (2014-07-09), XP055196354, Retrieved from the Internet: URL:http://www.healthline.com/health/chron ic-pain/treating-pain-with-heat-and-cold#H eatvs.Cold1 [retrieved on 2015-06-17] * page 1, paragraph 1 * | 1-15 | |

TECHNICAL FIELDS
SEARCHED (IPC)

G06F

-/--

The present search report has been drawn up for all claims

| Place of search | Date of completion of the search | Examiner |
|---|---|---|
| Munich | 19 June 2015 | Schmitt, Constanze |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 15 15 1523

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Craig Deneger ET AL: "How to use heat and cold to treat athletic injuries", Therapeutic Modalities for Muscoskeletal Injuries (Excerpt), 1 January 2010 (2010-01-01), XP055196561, ISBN: 978-0-73-607891-7 Retrieved from the Internet: URL:http://www.humankinetics.com/excerpts/ excerpts/how-to-use-heat-and-cold-to-treat -athletic-injuries [retrieved on 2015-06-17] * page 1 * | 1-15 | |
| A | Ian Janssen ET AL: "Body mass index and waist circumference independently contribute to the prediction of nonabdominal, abdominal subcutaneous, and visceral fat", The American Journal of Clinical Nutrition, 1 January 2002 (2002-01-01), pages 683-688, XP055196962, Retrieved from the Internet: URL:http://ajcn.nutrition.org/content/75/4 /683.full.pdf [retrieved on 2015-06-19] * abstract * * page 684, column 1, paragraph 3 - column 2, paragraph 1 * | 3,5-11 | |

TECHNICAL FIELDS SEARCHED (IPC)

The present search report has been drawn up for all claims

| Place of search | Date of completion of the search | Examiner |
|---|---|---|
| Munich | 19 June 2015 | Schmitt, Constanze |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100319110 A

**Non-patent literature cited in the description**

- **FIALA D ; LOMAS KJ ; STROHRER M.** A computer model of human thermoregulation for a wide range of environmental conditions: the passive system. *Journal of Applied Physiology,* 1999, 1957-1972
- Anthropometric Indices of Obesity and Regional Distribution of Fat Depots. **HAN TS ; LEAN MEJ.** International Textbook of Obesity. John Wiley & Sons Ltd, 2001, 51-65
- **KUK JL ; LEE SJ ; HEYMSFIELD SB ; ROSS R.** Waist circumference and abdominal adipose tissue distribution: influence of age and sex. *Am J Clin Nutr,* 2005, vol. 81, 1330-1334
- **WEISS WW ; CLARK FC.** Three protocols for measuring subcutaneous fat thickness on the upper extremities. *European Journal of Applied Physiology and Occupational Physiology,* 1987, vol. 56 (2), 217-221